# EUROPEAN PATENT APPLICATION

(11) **EP 2 192 408 A1**
(43) Date of publication of application: **02.06.2010**
(21) Application number: 09176689.9
(22) Date of filing: 20.11.2009
(51) Int. Cl.: G01N 33/53

(54) **Agents and methods for spectrometric analysis**

(30) Priority: 21.11.2008 US 275734
(71) Applicant: Morpho Detection, Inc., Newark, CA 94560 (US)
(72) Inventor: Pris, Andrew David, Clifton Park, NY 12065 (US); Paxon, Tracy Lynn, Waterford, NY 12188 (US); Wroczynski, Ronald James, Newark, CA 94560 (US); Murray, Anthony John, Newark, CA 94560 (US)
(74) Representative: Smaggasgale, Gillian Helen

(57) **Abstract**

Disclosed herein are agents, methods, and kits for determining the presence or concentration of a target, or multiple targets, in a sample, in a uniplexed or multiplexed fashion. In general, the methods enable the analysis of small molecules produced or consumed in liquid-phase that may be analyzed using gas or vapor phase detection methods.

## Description

### BACKGROUND

Provided herein are agents, methods, and devices for a uniplexed or multiplexed assays using gas and vapor phase analysis. More particularly, the present disclosure provides various combinations of catalysts and their associated substrates and products, which are useful for spectrometric analysis.

Gas and vapor phase analytical methods such as ion mobility spectrometry (IMS), ion mobility trap spectrometry (ITMS), mass spectrometry (MS), high-field asymmetric waveform ion mobility spectrometry (FAIMS), differential mobility spectrometry (DMS), and gas chromatography (GC) may be used to detect and identify chemicals such as explosives, drugs, toxic industrial materials, or chemical weapons.

Gas and vapor phase analytical methods may be used to indirectly detect agents that are not amenable to ionization by schematically coupling the non-ionizable agent to a chemical that may be ionized.

To enhance the ability of indirect detection methods, needs exist for agent sets with distinguishable components that may be used for spectrometric analysis using gas or vapor phase analysis devices,

### BRIEF SUMMARY

In one a method described herein of determining the presence of a target in a test sample comprises: (a) providing a test sample and a target in the test sample; (b) providing a capture agent capable of selectively binding to the target, wherein the capture agent is adhered to a solid support; (c) providing a binder of selectively binding to the target, wherein the binder is coupled to a catalyst; (d) contacting the test sample with the capture agent and the binder in an assay solution, wherein a captured target complex containing the capture agent, the target, and the binder is formed when the capture agent and the binder combine with the target; (e) separating the captured target complex from non-complexed assay components; (f) combining a substrate reactive with the catalyst to the separated captured target complex in solution, wherein the catalyst converts the substrate to a catalysis product; and (g) performing analysis of the solution of step (f) for an analyte selected from the substrate or the catalysis product; wherein the analyte is selected from 3,3',5,5'-tetramethylbenzidine (TMB), hydrogen peroxide, nicotinamide, 8-hydroxyquinoline, orthonitrophenol, paranitrophenol, phenol, pyridoxal, pyridoxamine, methyl salicylate, and ammonia.

In another aspect, a kit disclosed herein for determining presence of one or more targets in a test sample comprises a substrate and catalyst pair, wherein the substrate and the catalyst combine in a solution to produce a catalysis product, wherein only one of the substrate and the catalysis product is detectable using a preselected gas or ion spectrometric method.

In yet another aspect, provided herein are methods for determining the presence of multiple targets in a test sample comprising: (a) providing a test sample; (b) providing a plurality of capture agents adhered to a solid support, wherein each capture agent is capable of selectively binding to a preselected target from within the multiple targets; (c) providing a plurality of diverse binders each capable of selectively binding to a preselected target from within the multiple targets, wherein the diverse binders are coupled to preselected catalysts, respectively; (d) contacting the test sample the capture agents and the binders in an assay solution, wherein a heterogeneous population of captured target complexes containing the capture agents, corresponding preselected targets, and corresponding preselected binders are formed when the capture agents and the binders selectively bind with corresponding preselected targets present in the test sample; (e) washing the solid support to separate captured target complexes from non-complexed assay components; (f) combining a plurality of substrates reactive with the corresponding preselected catalysts to the separated captured target complexes in a solution, wherein the catalysts convert corresponding preselected substrates to catalysis products, respectively; and (g) performing analysis of the solution of step (f) for a plurality of diverse analytes selected from the substrates or the catalysis products; wherein the analytes are selected from the substrate or the catalysis product; wherein the analyte is selected from 3,3',5,5'-tetramethylbenzidine (TMB), hydrogen peroxide, nicotinamide, 8-hydroxyquinoline, orthonitrophenol, paranitrophenol, phenol, pyridoxal, pyridoxamine, methyl salicylate, and ammonia.

In yet another aspect, a method disclosed herein of determining presence of a target in a test sample comprising: (a) providing a test sample and a target present in the test sample; (b) providing a capture agent capable of selectively binding to the target, wherein the capture agent is adhered to a superparamagnetic bead; (c) providing a binder capable of selectively binding to the target, wherein the binder is coupled to a catalyst; (d) contacting the test sample with the capture agent and the binder in an assay solution, wherein a captured target complex containing the capture agent, the target, and the binder is formed when the capture agent and the binder combine with the target present in the test sample; (e) washing the solid support to separate the captured target complex from non-complexed assay components; (f) combining a substrate reactive with the catalyst to the separated captured target complex in a solution, wherein the catalyst converts the substrate to a catalysis product; and (g) performing analysis of the solution of step (f) for an analyte selected from the substrate or the catalysis product; wherein the analyte is selected from 3,3',5,5'-tetramethylbenzidine (TMB), hydrogen peroxide, nicotinamide, 8-hydroxyquinoline, orthonitrophenol, paranitrophenol, phenol, pyridoxal, pyridoxamine, methyl salicylate, and ammonia.

In yet another aspect, a method described herein of determining the presence of a target in a test sample comprises: (a) providing a test sample and a target present in the test sample; (b) providing a capture agent capable of selectively binding to the target, wherein the capture agent is adhered to a solid support; (c) providing a binder capable of selectively binding to the target, wherein the binder is coupled to a catalyst; (d) contacting the test sample with the capture agent and the binder in an assay solution, wherein a captured target complex containing the capture agent, the target, and the binder is formed when the capture agent and the binder combine with the target present in the test sample; (e) washing the solid support to separate the captured target complex from non-complexed assay components; (f) combining a substrate reactive with the catalyst to the separated captured target complex in a solution, wherein the catalyst converts the substrate to a catalysis product; and (g) performing analysis of the solution of step (f) for an analyte selected from the substrate or the catalysis product; wherein the analyte is selected from 3,3',5,5'-tetramethylbenzidine (TMB), hydrogen peroxide, nicotinamide, 8-hydroxyquinoline, pyridoxal, and pyridoxamine.

### DETAILED DESCRIPTION

To more clearly and concisely describe and point out the subject matter of the claimed invention, the following definitions are provided for specific terms, which are used in the following description and the appended claims.

The singular forms "a" "an" and "the" include plural referents unless the context clearly dictates otherwise. Approximating language, as used herein throughout the specification and claims, may be applied to modify any quantitative representation that could permissibly vary without resulting in a change in the basic function to which it is related. Accordingly, a value modified by a term such as "about" is not to be limited to the precise value specified. Unless otherwise indicated, all numbers expressing quantities of ingredients, properties such as molecular weight, reaction conditions, so forth used in the specification and claims are to be understood as being modified in all instances by the term "about." Accordingly, unless indicated to the contrary, the numerical parameters set forth in the following specification and attached claims are approximations that may vary depending upon the desired properties sought to be obtained by the present invention. At the very least each numerical parameter should at least be construed in light of the number of reported significant digits and by applying ordinary rounding techniques.

The term "analyte" as used herein generally refers to the assay component that is spectrometrically measured using the methods of the invention. Thus, in some embodiments the analyte may be the reaction substrate. In alternative embodiments, the analyte may be the catalysis product.

As used herein the term "catalyst" generally refers to substances that alter the rate of a chemical reaction without itself being consumed. The catalyst may either create or suppress the detectable molecule for the gas phase analysis. Non-limiting examples of catalyst include inorganic, organic or biological catalysts that effect redox, electronic or enzymatic conversion. Proton acids may be used for hydrolysis reactions. Multifunctional solids such as zeolites, alumina, graphitic carbon, and transition metals catalyze redox reactions (e.g., oxidation and hydrogenation). Biocatalysts such as enzymes, abzymes, ribozymes, and synthetic deoxyribozymes that transform biological substrates to catalysis products are also useful for the inventive methods.

As used herein, the term "detectable analyte" or "detectable species" or "detectable molecule" refers to an analyte that, when present in the sample or results from the catalysis reaction, is ionized and then undergoes ion motion in the established electromagnetic field that is associated with diffusion processes, gas density, ion-neutral interactions, and the electric field parameters.

As used herein, the term "ionizable analyte" refers to neutral atoms or molecules that lose or gain electrons, thereby acquiring a net charge, The ionizable analytes may be the reaction substrate or the catalysis product. The analytes should possess a gas phase ionization energy below the energy emitted by the source. Ionization sources can be broadly classified into two types: gas phase and desorption. Gas phase ionization may be accomplished using electron impact, chemical ionization, field ionization and photoionization; while desorption includes field desorption, electrospray, matrix-assisted desorption/ionization, plasma desorption, fast atom bombardment, secondary ion, and thermospray. Gas phase ionization is preferred for IMS analysis and is usually capable of ionizing molecules that possess an ionization energy below the source, have a boiling point below 500°C and have a molecular weight below 1000 Daltons.

The terms "sample" and "test sample" as used herein refer to any material that may contain a target for detection or quantification. The target may include an epitope or a reactive group (e.g., a group through which a compound of the invention can be conjugated to the target). The sample may also include diluents, buffers, detergents, and contaminating species, and debris. Samples may also include inorganic or organic molecules, nucleic acid polymers, nucleotides, oligonucleotides, peptides, and buffer solutions.

As used herein, the term "specific binding" refers to the specific recognition of one of two different molecules for the other compared to substantially less recognition of other molecules. The molecules may have areas on their surfaces or in cavities giving rise to specific recognition between the two molecules arising from one or more of electrostatic interactions, hydrogen bonding, or hydrophobic interactions. Specific binding examples include, but are not limited to, anlibody-antigen interactions, enzyme-substrate interactions, avidin-biotin interactions, or polynucleotide interactions. In some embodiments, a binder molecule may have an intrinsic equilibrium association constant (Ka) for the target no lower than about 10⁵ M⁻¹ under ambient conditions such as a pH of about 6 to about 8 and temperature ranging from about 0°C to about 37°C.

As used herein, the term "substrate" refers to the starting form of the molecule that the catalyst converts into the catalysis product.

As used herein, the term "target" refers to the component of a sample that may be detected when present in a sample, such as a biological sample. Representative biological targets may include one or more of natural or modified tissues, cells, organisms, peptides, proteins (e.g., antibodies, affibodies, or aptamers), nucleic acids (e.g., polynucleotides, DNA, RNA, or aptamers); polysaccharides (e.g., lectins or sugars), lipids, enzymes, enzyme substrates, ligands, receptors, antigens, and haptens. Representative small chemical molecule targets include pharmaceuticals, toxic industrial chemicals, toxic industrial materials, explosives, and their environmental or metabolic degradation products.

Disclosed herein are agents, methods, and kits for determining the presence or concentration of a target, or multiple targets, in a sample, in a uniplexed or multiplexed fashion. In general, the methods enable the analysis of small molecules produced or consumed in liquid-phase that may be analyzed using gas or vapor phase detection methods.

The present methods include substrate, catalyst, and catalysis-product sets that enable analysis of a sample for a target of interest. The catalyst may be associated with a binder. Either sequentially or simultaneously the sample is exposed to the catalyst associated target binder as well as to a solid support associated target capture agent. Binding the solid support-capture agent and the binder-catalyst to a target present in a sample followed by contacting the solid support-capture agent-target-binder-catalysis complex with a substrate that the catalyst is specific for under conditions that result in the genesis of the catalysis product. This solution is then spectrometrically analyzed for the change in the presence of the analyte, which may be the substrate or the catalysis product.

In some embodiments, the catalyst is linked to the binder before the initial association step. In alternative embodiments, the catalyst is coupled to a secondary binder that complexes with the binder associated with the target (e.g., via an anti-goat antibody that is reactive to goat anti-target antibody that has complexed with the target) following the initial association step.

In one embodiment, the substrate and the catalysis products are selected such that only one member of the substrate-catalysis product pair is detectable using ion mobility spectrometry. Thus, in some preferred embodiments, only the substrate is detectable using IMS. In other alternative preferred embodiments, only the catalysis product is detectable using ion mobility spectrometry.

The methods of the invention include a capture step in which the target capture agent is contacted with the sample. The capture agent has a binding affinity that enables specific binding between the capture agent and the target to form a capture agent-target complex. In some embodiments, the capture agent may be adhered to a solid support prior to the contacting step. In some alternative embodiments, the capture agent is adhered to a solid support following contacting and binding steps. During the capture step, the target is removed from the solution upon binding to the capture agent.

Then either following or concurrent with the capture step, the target is also contacted with a binder coupled to the catalyst, which forms a captured target complex.

Following the capture step, the captured target complex may be concentrated by an optional wash step. When the complex is adhered to the solid support, the washing and concentrating steps are accomplished by applying a wash solution to the container holding the complex adhered to the solid support. In alternative embodiments, the complex adhered to the solid support is removed from the solution. For example, when the solid support comprises a superparamagnetic bead, the superparamagnetic bead may be restrained by application of a magnetic field and a wash solution applied. In all embodiments, the wash solution may be removed by aspiration or decantation.

The catalysis step results when the catalyst and the substrate combine to generate the catalysis product. The analyte, which may be the substrate or the catalysis product, is ionized by the gas or vapor phase analytical device.

Small molecules adjusted by the liquid phase assay can be analyzed using other types of vapor or gas phase analysis including, but not limited to, ion trap mobility spectrometry, differential mobility spectrometry, field asymmetric ion mobility spectrometry, aspiration ion mobility spectrometry, mass spectrometry, gas chromatography, spectroscopy and other analytical methods that combine selective analysis compounds and mass, electronic, optical or thermal transduction.

The detection methods provided herein may be used for ion mobility spectrometry (IMS) or other spectrometric detection methods such as ion mobility spectrometry (IMS), ion mobility trap spectrometry (ITMS), mass spectrometry (MS), high-field asymmetric waveform ion mobility spectrometry (FAIMS), differential mobility spectrometry (DMS), and gas chromatography (GC).

In IMS, the analyte (i.e., the substrate or the catalysis product) present in either a gas or vapor state is ionized (e.g., using low-energy beta particles). The resulting ions must maintain their charge through gas phase ion-neutral interactions as they are manipulated by an electric field and the differential migration of the gas phase ions is measured.

The methods may be applied to any liquid phase assay that employs a capture agent and binder with known target selectivity and that is operated in a competitive or non-competitive fashion, where the assay outcome results in a change in the amount of the detectable product present, either through production or consumption of the detectable product, that is dependent, upon the presence or concentration of the target.

The detection step may qualitatively or quantitatively measure the presence or amount of the substrate or the catalysis products. The presence, absence, or amount of target present in the sample may be determined by spectrometric analysis of the sample. In general, a reaction scheme may be described by reactants transformed into products. The progression of the reaction, wherein the substrates are consumed and the products evolve, may be determined by observing or measuring the concentration of the substrate, the product, or both the substrate and the products. Where the target is present in the sample, catalysis products will be generated by the action of the catalyst. Where the target is absent from the sample, the catalyst will not be adhered to the solid support and the catalysis product will not be generated and not detected.

The catalyst may work to either create the detectable species from a non-detectable form (e.g., hydrolysis of a sugar group from the non-detectable species as with a galactosidase) or alternatively create a non-detectable form from the detectable form (e.g., creation of a radical group that polymerizes or combines two molecule of the detectable form as with a peroxidase). In each of the cases the gas phase analysis device will monitor the creation or reduction of the detectable species and relate that to either the presence (qualitative) or amount (quantitative) of the catalyst present. Representative detectable species include pyridoxamine, pyridoxal (by negative ion or positive ion sensitive analyzers), and hydrogen peroxide, 3,3',5,5'-tetramethylbenzidine (TMB), nicotinamide, 8-hydroxyquinoline (by positive ion sensitive analyzers).

The detectability of exemplary substrates is listed below in Table 1. the detectability of exemplary catalysis products is listed below in stable 2.

**Table 1**

| **Substrate** | **Detectable** |
|---|---|
| 3,3',5,5'-tetramethylbenzidine (TMB) | Yes |
| 3-cyanopyridine | No |
| 8-hydroxyquinoline glucopyranoside | No |
| 8-hydroxyquinoline glucuronide | No |
| 8-hydroxyquinoline β-D-galactopyranoside | No |
| glucose | No |
| hydrogen peroxide | Yes |
| orthonitrophenylgalactoside | No |
| orthonitrophenylglucopyranoside | No |
| paranitrophenol phosphate | No |
| phenylphosphate | No |
| pyridoxal phosphate | No |
| pyridoxamine phosphate | No |
| methyl salicylate glucuronide | No |
| urea | No |

**Table 2**

| **Catalysis Product** | **Detectable** |
|---|---|
| 8-hydroxyquinoline | Yes |
| ammonia | Yes |
| hydrogen peroxide | Yes |
| methyl salicylate | Yes |
| nicotinamide | Yes |
| orthonitrophenol | Yes |
| paranitrophenol | Yes |
| phenol | Yes |
| pyridoxal | Yes |
| pyridoxamine | Yes |
| TMB reaction product | No |
| water | No |

The sample in the provided assays may be of any source, for a biological sample. A biological sample may be of prokaryotic origin or eukaryotic origin. Suitable targets for use in the liquid phase assay include living targets and non-living targets. Examples of targets include, but are not limited to, prokaryotic cells, eukaryotic cells, bacteria, viruses, proteins, polypeptides, toxins, liposomes, particles, ligands, amino acids, nucleic acids, hormones, pharmaceuticals, toxic industrial chemicals, toxic industrial materials individually or in any combinations thereof. The target includes extracts of the above living or non-living targets.

In some embodiments, the target is attached to a solid support through a capture agent (e.g., an antibody, an aptamer, an affibody, or a ligand). A binder with an affinity for the target is coupled to the catalyst. In some embodiments, the binder is the same chemical species as the capture agent (e.g., a second antibody molecule with the same amino acid sequence as the capture agent). In alternative embodiments, the binder is different from the capture agent (e.g., an antibody with a different amino acid sequence or an aptamer). In all embodiments, both the capture agent and the binder is capable of specifically binding to the target. Suitable binders may include one or more of natural or modified peptides, proteins (e.g., antibodies or affibodies), nucleic acids (e.g., polynucleotides, DNA, RNA, or aptamers); polysaccharides (e.g., lectins, sugars), lipids, enzymes, enzyme substrates or inhibitors, ligands, and receptors.

In the assays, the target is adhered to a solid support, which may be any surface comprised of a porous or non-porous water-insoluble material. In some embodiments, the end user performs that step of adhering the target or a capture binder for the analyte to the solid surface. The surface can have any one of a number of shapes, such as a plate, a well, a strip, a rod, a particle, or a bead. The surface can be hydrophilic or capable of being rendered hydrophilic and includes inorganic powders such as silica, magnesium sulfate, and alumina, natural polymeric materials, such as materials derived from cellulose, such as fiber containing papers (e.g., filter paper or chromatographic paper). The solid support may comprise synthetic or modified naturally occurring polymers, such as nitrocellulose, cellulose acetate, poly(vinyl chloride), dextran, polyacrylate, polyethylene, polypropylene, poly(4-methylbutene), polystyrene, polymethacrylate, poly(ethylene terephthalate), nylon, or poly(vinyl butyrate). In embodiments where the analyte is vaporized while attached to the solid support, the solid support is preferably made of a non-volatile material such as a metal.

Solid supports suitable for use in the present invention are typically substantially insoluble in liquid phases. Various supports are available and are known to one of ordinary skill in the art. Solid supports may include solid and semi-solid matrixes, such as aerogels, hydrogels, beads, biochips (including thin film coated biochips), microfluidic chip, silicon chip, multi-well plates (also referred to as microtitre plates or microplates), membranes, conducting and nonconducting metals, glass (including microscope slides) and magnetic supports. More specific examples of useful solid supports include polymeric membranes, particles, derivatized plastic films, glass beads, cotton, plastic beads, alumina gels, polysaccharides such as poly(acrylate), polystyrene, polyol, cellulose, dextran, starch, ficoll, heparin, glycogen, amylopectin, mannan, inulin, nitrocellulose, diazocellulose, polyvinylchloride, polypropylene, polyethylene (including poly(ethylene glycol)), nylon, polyvinylidene, polyethersulfone, latex bead, magnetic bead, paramagnetic bead, superparamagnetic bead, and starch. Multiwell plates enable high throughput analyses. Lateral flow membranes facilitate the separation/wash step. Solid supports in the form of beads or particles increased reaction kinetics of both the capture and catalysis steps.

In some embodiments, the solid support may comprise a magnetic or paramagnetic or superparamagnetic particle or bead, e.g., iron (Fe), cobalt (Co), or nickel-iron alloys. These magnetic or paramagnetic or superparamagnetic particles may also comprise nonmagnetic materials such as polystyrene in which superparamagnetic subparticles (e.g., iron oxide particles) are embedded. A solid support may also include a detectable material such as a dye, a colorant, a hybridization tag or have a specific refractive index so that the particle may be visually detected on the sample and identified among other particles as well as the solution.

The methods provided herein include a catalysis step, in which a catalyst converts a substrate associated with a target to a catalysis product. As used herein the term "catalyst" generally refers to substances that alter the rate of a chemical reaction without itself being consumed. The catalyst may either create or suppress the detectable molecule for the gas phase analysis. Non-limiting examples of catalysts include inorganic, organic or biological catalysts that allow for redox, electronic or enzymatic conversion. The catalyst may be selected according to the chemical reaction in which a selected substrate is converted into catalysis product. Proton acids may be used for hydrolysis reactions. Multifunctional solids such as zeolites, alumina, graphitic carbon, and transition metals catalyze redox reactions (e.g., oxidation and hydrogenation). Biocatalysts such as enzymes, abzymes, ribozymes, and synthetic deoxyribozymes transform biological substrates to catalysis products.

In some embodiments, a catalyst is covalently bound to the binder. In some alternative embodiments, the catalyst is covalently bound to a secondary binder. Exemplary secondary binders include antibodies or other binding agents that selectively bind a portion of the primary binder or a target epitope. This binder can be the same type or different type from the capture agent associated with the solid support.

The catalyst may work to either create the detectable species from a non-detectable form (e.g., hydrolysis of a sugar group from the non-detectable species as with a galactosidase) or alternatively create a non-detectable form from the detectable form (e.g., creation of a radical group that polymerizes or combines two molecules of the detectable form as with a peroxidase). In each of the cases the gas phase analysis device will monitor the creation or reduction of the detectable species and relate that to either the presence or amount of the catalyst present.

Exemplary substrate, catalyst, product combinations useful for the inventive methods are set out in Table 3 below.

**Table 3**

| **Substrate** | **Catalyst** | **Product** |
|---|---|---|
| 3,3',5,5'-tetramethylbenzidine (TMB) | peroxidase | TMB reaction product |
| 3-cyanopyridine | nitrile hydratase | nicotinamide |
| 8-hydroxyquinoline glucuronide | glucuronidase | 8-hydroxyquinoline |
| 8-hydroxyquinoline glucopyranoside | glucosidase | 8-hydroxyquinoline |
| 8-hydroxyquinoline β-D-galactopyranoside | galactosidase | 8-hydroxyquinoline |
| glucose | glucose oxidase | hydrogen peroxide |
| hydrogen peroxide | catalase | water |
| orthonitrophenylgalactoside | galactosidase | orthonitrophenol |
| orthonitrophenylglucopyranos ide | glucosidase | orthonitrophenol |
| paranitrophenol phosphate | alkaline phosphatase | paranitrophenol |
| phenylphosphate | alkaline phosphatase | phenol |
| pyridoxal phosphate | alkaline phosphatase | pyridoxal |
| pyridoxamine phosphate | alkaline phosphatase | pyridoxamine |
| methyl salicylate glucuronide | glucuronidase | methyl salicylate |
| urea | urease | ammonia |

In some embodiments, a non-ionic detergent may be added to the sample. Generally the detergent will be present in from about 0.01 to 0.1 percent volumes. Illustrative non-ionic detergents include the polyoxyalkylene diols, for example, Pluronics, Tweens, or Triton X-1 00.

Although reaction times vary based on the temperature, concentrations of target and capture agent, or catalyst substrate respectively, typical reaction times for each individual reaction steps fall between 2 and 180 minutes. When the Components of the invention are species that bind to targets (e.g., capture agents, enzymes, receptors, ligands, antigens, or the reaction time between the compound or conjugate of the invention and the target will usually be at least about 2 minutes, more usually at least about 30 minutes and preferably not more than about 180 minutes. By using a specific time period for the reaction or taking aliquots at 2 different times, the rate of reaction can be determined for comparison with other determinations. The temperature will generally be in the range of about 20°C to 50°C, more usually in the range of about 25°C to 40°C.

For embodiments in which the methods include the step of adhering the capture agent to the solid support, binding sites on the solid support may first be blocked with a suitable blocking agent, e.g., casein.

In the non-competitive assay form, the assay labels the solid support captured target with a catalyst that converts a molecule either into a form that is detectable, or not detectable, by IMS. In the competitive assay form, the assay displaces catalyst labels from the support or the catalyst must compete with the target to bind to the support where the bound catalyst converts a molecule either into a form that is detectable, or not detectable, by a gas phase analysis.

The assays may further include one or more control steps where a sample known to contain the target (positive control) is analyzed in parallel or in series with the sample. Similarly, the assays may further include one or more control steps where a sample known not to contain the target (negative control) is analyzed in parallel or in series with the sample.

In addition to the samples to be tested, a series of wells may be prepared using known concentrations of the analyte. A curve, plotting the detected measurements versus the known concentration of analyte in these standard wells is prepared. By comparing the detected measurements of the samples to this standard curve, the concentration of the analyte in the unknown samples may then be determined. Alternatively, the standard curve can be achieved through standard additions.

The analyte may be ionized using any art-recognized ionization method, such as chemical ionization or electron ionization. In chemical ionization, ions are produced through the collision of the analyte of ions of a reagent gas in the ion source. The reagent gases are converted to plasma by electron bombardment to create ionization plasma. Reactions between the analyte and the plasma form positive and negative ions.

An aliquot of the sample including the complexed solid support, noncomplexed solid support, unreacted substrate and the catalysis product are introduced into the vapor phase spectrometer. The sample is ionized, then the detectable substrate, the detectable product, or both detectable substrate and the detectable product is observed and identified via a previously established chemical library.

Gas phase ion spectrometers include an ion source that supplies gas phase ions. Gas phase ion spectrometers include, for example, mass spectrometers, ion mobility spectrometers, ion trap mobility spectrometers, differential mobility spectrometers, field asymmetric ion mobility spectrometer, aspiration ion mobility spectrometers and total ion current measuring devices. In one embodiment, an IMS is used to detect and characterize the detectable product of the assay. The solid supports and the substrate in the liquid phase are placed within the IMS and heated to a temperature from about 25°C to about 600°C depending on the detectable molecule, e.g., the product produced by the enzyme-substrate reaction where the substrate by itself is not detectable.

The detectable species provided herein are useful for multiplex assays in addition to uniplex assays. Consequently, for IMS that are run within their normal operating parameters, which includes the presence of the negative mode reactant ion, chloride ion and the positive mode reactant ion, ammonia, yields distinguishable species in the negative mode of IMS and also provides multiple distinguishable species in the positive mode of IMS, The combination of substrates and associated ionizable products enables multiplexing assays where multiple distinguishable species whose presences is affected through the assay in a manner that can be related to a specific target can be accomplished not only through each distinguishable species having a unique IMS mobility but also the mode in which the species has a mobility (i.e., positive or negative mode). In multiplex assays the substrate and catalyst pairs are selected so that multiple analytes are detectable. The multiple capture agents (i.e., C₁, C₂, C₃, ... Cₙ₎ are selected to specifically bind to putative targets (Ti, T₂, T₃, ... Tₙ₎for which the sample is interrogated. Also, the multiple binders (i.e., B₁, 8₂, B₃, ... Bₙ₎ are also selected to specifically bind to putative targets (T₁, T₂, T₃, ... Tₙ₎for which the sample is interrogated. When a target (T₁) present in the sample is bound both by C₁ and B₁ a captured target complex is formed. In some multiplexed applications, both the capture agents and the binders are selected to specifically bind a single putative target thought to be present in the sample. In such embodiments cross-reactivity among the non-corresponding capture agents, targets, and binders is disfavored. Similarly, in multiplexed applications the substrate and catalyst sets are paired to capture agents and binders selected for a single rather than multiplex putative targets facilitating clear correspondence of the analyte with the presence, absence, or quantity of the targets in the sample.

The substrates and/or catalysis products are present if the target of interest is present in the test sample and the amount of the substrates and/or catalysis products created/consumed depend upon the amount of analyte in the assay. Thus, the present methods may produce qualitative, quantitative, or both qualitative and quantitative information about the test sample.

Also provided are kits for the detection of a target analyte comprising one substrate and catalyst pair, or multiple substrate and catalyst pairs useful for the methods of the invention. Additional kit components may include a solid support, instructions to use the solid support, capture agents, binders, substrates, buffers and standards.

The kits may further include various for use in the inventive assays. These buffers include, but are not limited to, PBS, Tris, MOPS, HEPES, and phosphates allowing for of pH. Although pH may vary depending upon the particular assay, generally concentration of buffer may be in the range of about 0.1 mM to 500 mM. Alternatively, the concentration of the buffer may be in the of 0.5 mM to 200 mM.

The pH will vary depending upon the particular assay system, generally within a readily determinable range wherein the concentration of buffer is generally in the of about 0.1 to 50 mM, more usually 0.5 to 20 mM.

The kit reagents may be provided in solution form for ease of handling. Alternatively, one or more reagents may be lyophilized to preserve activity and extend shelf life. Additionally, compatible reagents (e.g., signal generator, buffer, and peroxide) may be combined in solution at concentrations that enable facile use of the kit components.

### EXAMPLES

Practice of the invention will be still more fully understood from the following examples, which are presented herein for illustration only and should not be construed as limiting the invention in any way.

In the following examples all buffers used were prepared in 18 MΩ Milli-Q water (Millipore, Billerica, MA). All samples were analyzed with an Itemiser^{3®} ITMS instrument (GE Security, Bradenton, FL), which was set in dual mode with a default sampling time of 7 seconds, a desorber temperature of 220°C and a detector temperature of 205°C. The Itemiser^{3®} was run with the semi-permeable membrane in place and with both the explosive (methlyene chloride) and narcotic reactant ion (ammonia) present Within the system. The chloride reactant ion in a reactant ion peak (RIP) at 3.19 ms in the negative and the ammonia in a RIP at 3.48 ms in the positive mode. These compounds were as being detected/non-detected on the VaporTracer^{2®} and MobileTrace^{®} ITMS systems.

Specifically, 10 µL of the solution to be analyzed was placed upon a woven polyamide gold sample trap (GE Security, Bradenton, FL) and immediately inserted into the sampling port of the instrument, which triggered the sample acquisition.

Itemiser software (version 8.12) was also used to extract the "Mean AHeight" of the peak of interest, from within the plasmagram. The peak of interest for each compound was determined through analysis of stock solutions of the product and substrate.

The modified ELISA non-competitive assays were run as follows. Goat anti-E. coli modified superparamagnetic particles (Invitrogen, Carlsbad, CA) were obtained and prepared according to instructions and diluted to 0.2X their original concentration in the appropriate buffer for the assay. Specifically, a 10 mM Trishydroxymethyl (aminomethane), 150 mM sodium chloride, 1 mM ZnCl₂, 1mM MgCl₂ (Sigma Aldrich, St. Louis, MO) (pH 8.0) buffer (Tris buffer) was used in the alkaline phosphatase (AP) assay and a 10 mM sodium phosphate, 137 mM sodium chloride (Sigma Aldrich, St. Louis, MO) (pH 7.4) (PBS buffer) was used in the glucuronidase assay. Goat anti-E. coli conjugated alkaline phosphatase (AP) was obtained from KPL (Baltimore, MD) in lyophilized form and diluted to 1 mg/mL concentration in the Tris buffer according to directions. Glucuronidase was obtained from Roche (Indianapolis, IN) in lyophilized form and conjugated to goat anti-E. coli (KPL, Baltimore, MD) with succinimidyl 4-formylbenzoate (SFB, Thermo Pierce, Rockford, IL) and succinimidyl 4-hydrazinonicotinate acetone hydrazone (SANH, Thermo Pierce, Rockford, IL) according to vendor instructions to produce a final antibody concentration of 0.3 mg/mL in the PBS buffer. E. coli target solutions were from a lyophilized heat E. coli standard (KPL, Baltimoro, MD) that was in water to 10⁹ CFU/mL. Subsequent dilutions were made from this stock solution.

The assays were run by placing 114 µL of the buffer (Tris for the AP reaction and for the glucuronidase reaction), 13 µL of the 0.2X goat anti-E. coli superparamagnetic particles (in the appropriate buffer), 12 µL of the enzyme modified goat-anti E. coli antibodies, and 10 µL of E. coli target solution into a 500 µL tube. The microcentrifuge tube was rocked for 5 min at room temperature. A sheathed rare-earth magnet was placed within this solution for 30 seconds to collect the superparamagnetic particles upon the sheath. The particles were removed from the assay solution, gently rinsed with the appropriate buffer, and redispersed within 100 µL of a 1 mg/mL enzyme substrate solution (in the appropriate buffer). This solution was heated at 37°C for 5 minutes. After this time, a 10-µL sample was analyzed by the ITMS as described above.

### Example 1: TMB/peroxidase/TMB polymer

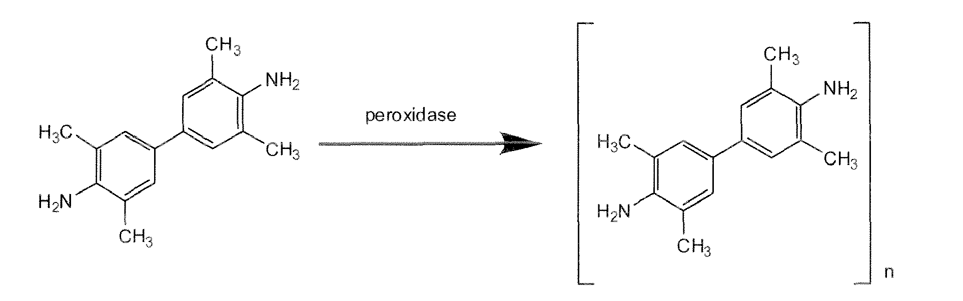

3,3',5,5'-tetramethylbenzidine (TMB) (0.5 mg/mL, Sigma Aldrich, St. Louis, MO) was prepared in 50 mM sodium phosphate, 0.05% H₂O₂ (Fisher Scientific, Pittsburgh, PA) (pH 5.0) buffer and analyzed via ITMS. The TOMB produces a positive mode ITMS peak at a calibrated drift time of 7.41 ms.

A 95 µL aliquot of this solution was mixed with 5 µL of horseradish peroxidase in buffer (HRP, Sigma-Aldrich, Saint Louis, MO) for a final HRP amount of 0.5 units. This solution was allowed to react for 1 minute at room temperature to create the TMB end polymer product. After this time, the reaction was sampled and immediately with ITMS, the TMB peak (7.41 ms) the positive mode plasmagram has been depleted by the enzymatic reaction.

| **Sample** | **Mean AHeight ITMS Signal** |
|---|---|
| | **(Pos.Mode. 7.41 ms) (Arb. Units)** |
| buffer (50 mM phosphate, 0.05% H2O2) | 0 |
| 0.5 mg/mL TMB in buffer | 647 |
| 0.5 unit HRP in 0.5 mg/mL TMB in buffer (1 min reaction time) | 0 |

### Example 2: 3-cyanopyridine / nitride hydratase / nicotinamide

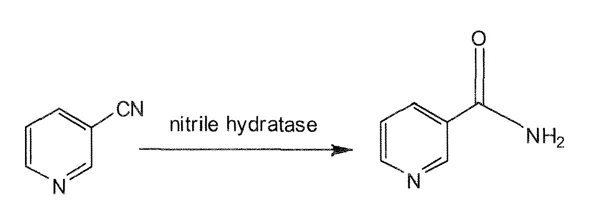

3-cyanopyridine (1 mg/mL, Sigma-Aldrich, St. Louis, MO) and 1 mg/mL of nicotinamide were prepared separately in 10 mM sodium phosphate, 137 mM sodium chloride (Sigma Aldrich, St. Louis, MO) (pH 7.4) buffer and analyzed via ITMS. The 3-cyanopyridine produces no discernable signal in the negative or positive mode of ITMS. The nicotinamide produces a peak using the positive mode at a calibrated drift time of 5.11 ms.

A 95 µL aliquot of the 3-cyanopyridine solution mixed with 5 µL of nitrile hydratase (Codexis, Redwood City, CA) in buffer for a final nitrile hydratase amount of 0.245 units. This solution was allowed to react for 5 minutes at 37°C to create the nicotinamide end product. After this time the reaction mixture was immediately sampled and analyzed with ITMS, where the nicotinamide peak (5.11 ms) from the positive mode plasmagram appeared due to the enzymatic reaction.

| **Sample** | **Mean AHeight ITMS Signal (Pos.** |
|---|---|
| | **Mode, 5.11 ms) (Arb. Units)** |
| 10 mM phosphate, 137 mM sodium chloride (pH 7.4) | 0 |
| 1 mg/ml nicotinamide in buffer | 9328 |
| 1 mg/mL 3-cyanopyridine in buffer | 1975 |
| 0.245 unit nitride hydratase in 1 in buffer (5 min reaction time) | 10060 |

### Example 3: pyridoxamine phosphate / alkaline phosphatase / pyridoxamine and pyridoxal phosphate / alkaline phosphatase /pyridoxal

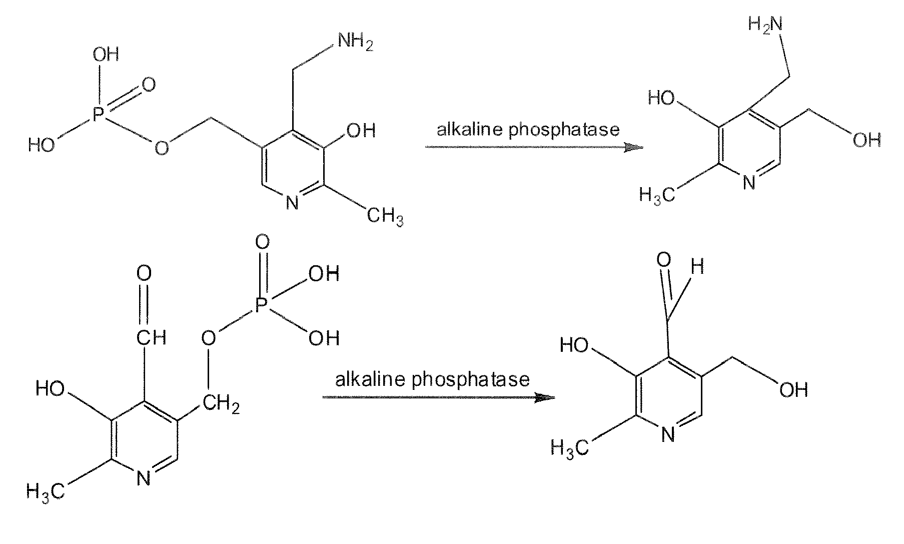

Pyridoxamine-5-phosphate, pyridoxamine, pyridoxal-5-phosphate, and pyridoxal (1 mg/mL. Sigma Aldrich, St. Louis, MO) were individually prepared in 10 mM Trishydroxymethyl (aminomethane) (Tris), 150 mM sodium chloride, 1 mM ZnCl₂, 1 mM MgCl₂ (Sigma Aldrich, St. Louis, MO) (pH 8.0) buffer and were all separately analyzed using ITMS. The pyridoxamine-5-phosphate and pyridoxal-5-phosphate produces no discernable signal within the negative or positive mode of ITMS. The pyridoxamine and pyridoxal samples both result in distinctive positive and negative mode peaks, but only the negative mode 5.86 ms and 5.63 ms peaks were monitored for pyridoxamine and pyridoxa!, respectively.

A 95 µL of the pyridoxamine-5-phosphate was mixed with 5 µL of alkaline phosphatase (AP, Sigma Aldrich, St. Louis, MO) in buffer for a final AP amount of 47 units. This solution was allowed to react for 15 min at 37 °C to the pyridoxamine end product followed by immediate ITMS analysis. The ITMS negative mode plasmagram of this enzymatic reaction disp!ays the pyridoxamine peak (5.86 ms).

| **Sample** | **Mean AHeight ITMS Signal** |
|---|---|
| | **(Nea.Mode, 5.86 ms) (Arb. Units)** |
| 10 mM Tris, 150 mM NaCl, 1 mM MgCl₂, 1mM ZnCl₂ (pH 8) | 0 |
| 1 mg/mL pyridoxamine in buffer | 172 |
| 1 mg/mL pyridoxamine-5-phosphate in buffer | 0 |
| 47 unit alkaline phosphatase in 1 mg/mL pyridoxamine-5-phosphate in buffer (15 min reaction time) | 275 |

Separately, a 95 µL aliquot of the pyridoxal-5-phosphate was mixed with 5 µL of alkaline phosphatase (AP, Sigma Aldrich, St. Louis, MO) in buffer for a final AP amount of 47 units. This solution was allowed to react for 5 min at 37°C to create the pyridoxal end product and immediately analyzed with ITMS. The negative mode ITMS plasmagram now displays the pyridoxal peak (5.63 ms) due to the enzymatic reaction.

| **Sample** | **Mean AHeight ITMS Signal** |
|---|---|
| | **(Neg.Mode, 5.63 ms) (Arb. Units)** |
| 10 mM Tris, 150 mM NaCl, 1 mM MgCl₂, 1mM ZnCl₂ (pH 8) | 0 |
| 1 mg/mL pyridoxal in buffer | 2228 |
| 1 mg/mL pyridoxal phosphate in buffer | 0 |
| 47 unit alkaline phosphatase in 1 mg/mL pyridoxal phosphate in buffer (5 min reaction time) | 2539 |

### Example 4: 8-hydroxyquinoline glucuronide / glucuronidase / 8-hydroxyquinoline and 8-hydroxyquinoline glucopyranoside / glucosidase / 8-hydroxyquinoline

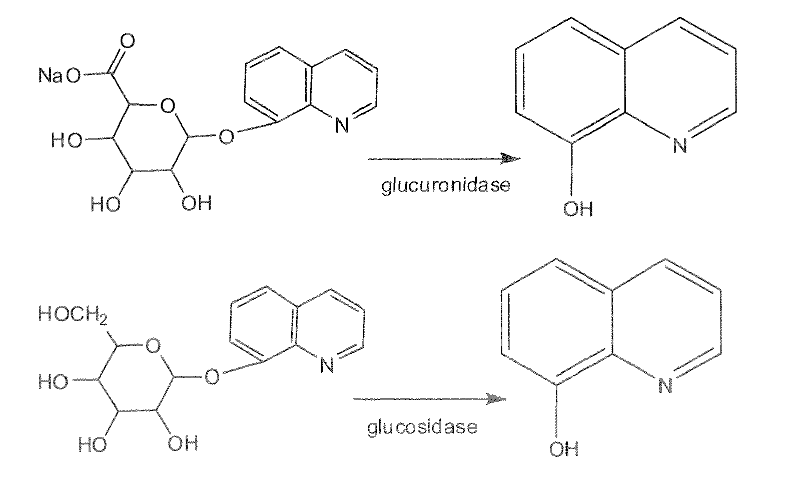

8-hydroxyquinoline glucopyranoside, 8-hydroxyquinoline glucuronide, and 8-hydroxyquinoline (1 mg/mL, Sigma Aldrich, St. Louis, MO) were individually prepared in 10 mM sodium phosphate, 137 mM sodium chloride (Sigma Aldrich, St. Louis, MO) (pH 7.4) buffer and were all separately analyzed with ITMS. The 8-hydroxyquinoline glucopyranoside and the 8-hydroxyquinoline glucuronide produce no discernable signal within the negative or positive mode of ITMS. The 8-hydroxyquinoline produces a peak within the positive mode at a calibrated drift time of 5.29 ms.

A 95 µL aliquot of the 8-hydroxyquinoline glucopyranoside was mixed 5 µL of glucosidase (Sigma Aldrich, St. Louis, MO) in buffer for a final glucosidase amount of 20 units. This solution was allowed to react for 5 min at 37°C to create the 8-hydroxyquinoline end product. After the reaction time, the sample was immediately analyzed with ITMS, where the 8-hydroxyquinoline peak (5.29 ms) from the positive mode plasmagram appeared due to the enzymatic reaction.

| **Sample** | **Mean AHeight ITMS Signal** |
|---|---|
| | **(Pos.Mode, 5.29 ms) (Arb. Units)** |
| 10 mM phosphate, 137 mM sodium chloride (pH 7.4) | 1643 |
| 1 mg/ml hydroxyquinoline in buffer | 11345 |
| 1 mg/mL 8-hydroxyquinoline-β-D-glucopyranoside in buffer | 0 |
| 20 unit β-D-glucosidase in 1 mg/mL 8-hydmxyquinoline-β-D-glucopyranoside in buffer (5 min reaction time) | 10998 |

Separately, a 95 µL aliquot of the 8-hydroxyquinoline-glucuronide was mixed with 5 µL of glucuronidase (Sigma Aldrich, St. Louis, MO) in buffer for a final glucuronidase amount of 20 units. This solution was allowed to react for 5 min at 37°C to create the 8-hydroxyquinoline end-product and immediately analyzed with ITMS, the 8-hydroxyquinoline peak (5.29 ms) from the positive mode plasmagram has now appeared due to the enzymatic reaction.

| **Sample** | **Mean AHeight ITMS Signal** |
|---|---|
| | **(Pos.Mode, 5.29 ms) (Arb. Units)** |
| 10 mM phosphate, 137 mM sodium chloride (pH 7.4) | 1643 |
| 1 mg/ml hydroxyquinoline in buffer | 11345 |
| 1 mq/mL 8-hydroxyquinoline-β-D-glucuronide in buffer | 1859 |
| 20 unit β-D-glucuronidase in 1 mg/mL 8-hydroxyquinoline-β-D-glucuronide in buffer (5 min reaction time) | 11485 |

### Example 5: ITMS analysis of a modified ELISA assay for E. coli employing production of pyridoxal from pyridoxal-5-phosphate and alkaline phosphatase modified goat anti-E. coli.

The assay was run using Tris (10 mM Trishydroxymethyl (aminomethane) (Tris), 150 mM sodium chloride, 1 mM ZnCl₂, 1mM MgCl₂ (Sigma Aldrich, St. Louis, MO) (pH 8.0)) as the appropriate buffer. The sample containing 10⁷ E. coli results in a distinctive peak at 5.63 ms in the negative mode as expected for pyridoxal. If the assay is run in an identical fashion but 10 µL of buffer is used instead of adding 10 µL of a sample containing E. coli, no enzyme is delivered to the final solution, thus none of the pyridoxal phosphate is converted to the pyridoxal and no pyridoxal signal is obtained.

| | **Mean AHeight ITMS Signal** |
|---|---|
| **Sample** | **(Neg.Mode, 5.63 ms) (Arb. Units)** |
| 1 mg/mL pyridoxal phosphate in buffer | 0 |
| Assay with 0 CFU/mL *E. coli* | 0 |
| Assay with 10^7 CFU/mL *E. coli* | 135 |

### Example 6: ITMS analysis of a modified ELISA assay for E. coli employing production of 8-hydroxyquinoline from 8-hydroxyquinoline glucuronide and glucuronidase modified goat anti-E. coli.

The assay was run using PBS (10 mM sodium phosphate 137 mM sodium chloride, (Sigma Aldrich, Saint Louis, MO) (pH 7.4)) as the buffer. The sample containing various concentrations of E. coli results in a distinctive peak at 5.29 ms in the positive mode as expected for 8-hydroxyquinoline. When the assay was run using 10 µL of buffer instead of adding 10 µL of a sample containing E. coli, no enzyme is delivered to the final solution, Thus none of the 8-hydroxyquinoline glucuronide is to the 8-hydroxyquinoline and no 8-hydroxyquinoline signal is obtained. Furthermore, increased amounts of E. coli result in an increased ITMS response, indicating that this scheme can be useful for quantitative analysis.

| **Sample** | **Mean AHeight ITMS Signal** |
|---|---|
| | **(Pos.Mode, 5.29 ms) (Arb. Units)** |
| 1 mg/mL 8-hydroxyquinoline-β-D-glucuronide in buffer | 398 |
| Assay with 0 CFU/mL *E. coli* | 1450 |
| Assay with 10^⁶ CFU/mL *E. coli* | 2182 |
| Assay with 10^⁷ CFU/mL *E. coli* | 6015 |
| Assay with 10^⁸ CFU/mL *E. coli* | 5825 |

### Example 7: ITMS analysis of multiplexed creation of two distinct detectable products from within a single solution by producing 8-hydroxyquinoline from 8-hydroxyquinoline glucuronide and glucuronidase and by producing orthonitrophenol from orthonitrophenylgalactopyranoside and galactosidase.

The enzymatic reactions were carried out in a 10 mM sodium phosphate, 137 mM sodium chloride (Sigma Aldrich, St. Louis, MO) (pH 7.4) (PBS buffer). β-glucuronidase and β-galactosidase were obtained from Roche (Indianapolis, IN) in lyophilized form and each were diluted to a stock concentration of -1000 units/mL in PBS buffer. The reaction solution contained both 8-hydroxyquinoline-β-D-glucutoglucuronide and ortho-nitrophenyl-β-D-galactopyranoside each at a 1 ma/ml concentration in the PBS buffer.

Four different 100-βL solutions were created in the reaction solution to examine this multiplexed ability: no enzyme; 20 unit/mL β glucuronidase; 20 unit/mL β -galactosidase; 20 unit/mL β-glucuronidase and 20 unit/mL β-galactosidase.

Each of these solutions was rocked at room temperature for 5 minutes and 10 µL samples were with the ITMS in dual mode as described above. The positive and negative mode plasmagram collected from this single sample was saved and examined to extract the "Mean AHeight" for the o-nitrophenol (ONP) peak in the negative at a calibrated drift time of 5.09 ms and for the 8-hydroxyquinoline (8-HQ) in the positive mode at a calibrate drift time of 5.29 ms,

| **Sample** | **Mean AHeight ITMS Signal** | **Mean Height ITMS Signal** |
|---|---|---|
| | **(Neg.Mode, 5.09 ms) (Arb. Units)** | **(Pos.Mode, 5.29 ms) (Arb. Units)** |
| 1 mg/mL 8-hydroxyquinoline-β-D-glucuronide and 1 mg/mL ortho-nitrophenyl-β-D-galactopyranoside in buffer (5 min reaction time) | 0 | 148 |
| 20 unit β-D-gtucuronidase in 1 mg/mL 8-hydroxyquinoline-β-D-glucuronide and 1 mg/mL ortho-nitrophenyl-β-D-galactopyranoside in buffer (5 min reaction time) | 2565 | 10453 |
| 20 unit β-D-galactosidase in 1 mg/mL 8-hydroxyquinoline-β-D-glucuronide and 1 mg/mL ortho-nitrophenyl-β-D-galactopyranoside in buffer (5 min reaction time) | 7594 | 260 |
| 20 unit β-D-glucuronidase and 20 unit β-D-galactosidase in 1 mg/mL 8-hydroxyquinoline-β-D-glueuronide and 1 mg/mL ortho-nitrophenyl-β-D-galactopyranoside in buffer (5 min reaction time) | 7310 | 10313 |

### EQUIVALENTS

While embodiments of the invention have been described with reference to exemplary embodiments, it will be understood by those skilled in the art that various changes can be made and equivalents can be substituted for elements thereof without departing from the scope of the embodiments of the invention. In addition, many modifications can be made to adapt a particular situation or material to the teachings of embodiments of the invention without departing from the essential scope thereof. Therefore, it is intended that the embodiments of the invention not be limited to the particular embodiment disclosed as the best mode contemplated for carrying out this invention, but that the embodiments of the invention will include all embodiments falling within the scope of the appended claims.

## Claims

1. A method of determining the presence of a target in a test sample comprising:
(a) providing a test sample and a target present in the test sample;
(b) providing a capture agent capable of selectively binding to the target, wherein the capture agent is adhered to a solid support;
(c) providing a binder capable of selectively binding to the target, wherein the binder is coupled to a catalyst;
(d) contacting the test sample with the capture agent and the binder in an assay solution, wherein a captured target complex containing the capture agent, the target, and the binder is formed when the capture agent and the binder combine with the target;
(e) washing the solid support to separate captured target complex from non-complexed assay components;
(f) combining a substrate reactive with the catalyst to the separated captured target complex in solution, wherein the catalyst converts the substrate to a catalysis product; and
(g) performing analysis of the solution of step (f) for an analyte selected from the substrate or the catalysis product; wherein the analyte is selected from 3,3',5,5'-tetramethylbenzidine (TMB), hydrogen peroxide, nicotinamide, 8-hydroxyquinoline, pyridoxal, and pyridoxamine.

2. The of Claim 1, further comprising a step of ionizing components of the solution of step (f) before step (g).

3. The method of Claim 2, wherein the analyte is ionized using chemical, electrical, or photo ionization.

4. The method of any one of Claim 1 to 3, wherein the substrate is selected from is a 3,3',5,5'-tetramethylbenzidine, 3-cyanopyridine, 8-hydroxyquinoline glucopyranoside, 8-hydroxyquinoline glucuronide, 8-hydroxyquinoline β-D-galactopyranoside, pyridoxal phosphate, and pyridoxamine phosphate.

5. The method of Claims 1 to 4, wherein the capture agent is selected from an antibody, an aptamer, an affibody, and a ligand.

6. The method of any one of Claims 1 to 5, wherein the binder is selected from an antibody, an aptamer, an affibody, and a ligand.

7. The method of any one of Claims 1 to 6, wherein the solid support is selected from superparamagnetic particles, membranes, and polymer substrates.

8. The method of Claim 7, wherein the solid support is selected from synthetic or modified naturally occurring polymers, comprising nitrocellulose, cellulose acetate, poly(vinyl chloride), dextran, polyacrylate, polyethylene, polyethersulfone, polypropylene, poly(4-methylbutene), polystyrene, polymethacrylate, poly(ethylene terephthalate), nylon, or poly(vinyl butyrate).

9. The method any one of Claims 1 to 8, wherein the solid support comprises a plate, a well, a strip, a rod, or a particle.

10. The method of any one of Claims 1 to 8, wherein the solid support is a membrane and the step (e) results from lateral flow of the non-complexed assay components across the membrane.

11. The method of any one of Claims 1 to 9, wherein step (g) includes determining the absence or presence of the analyte in the test sample.

12. The method of Claim 11, wherein the absence or presence of the analyte in the test sample is correlated with the absence or presence of the target in the test sample.

13. The method of any one of Claims 1 to 12, wherein the step (g) further includes quantifying an amount of the analyte in the test sample.

14. The method of Claim 13, wherein the amount of the analyte detected in the test sample is correlated with quantity of the target in the test sample.

15. The method of any one of Claims 1 to 14, wherein the target is selected from prokaryotic cells, eukaryotic cells, bacteria, viruses, proteins, polypeptides, toxins, liposomes, particles, ligands, amino acids, nucleic acids, hormones, pharmaceuticals, toxic industrial chemicals, toxic industrial materials, or combinations thereof.

16. The method of any one of Claims 1 to 15, wherein step (g) is performed using a method selected from ion mobility spectrometry (IMS), ion mobility trap spectrometry (ITMS), mass spectrometry (MS), high-field asymmetric waveform ion mobility spectrometry (FAIMS), differential mobility spectrometry (DMS), and gas chromatography (GC).

17. A kit for determining presence of one or more targets in a test sample comprising a substrate and catalyst pair, wherein the substrate and the catalyst combine in a solution to produce a catalysis product, wherein only one of the substrate and the catalysis product is detectable using gas or ion spectrometric methods.

18. The kit of claim 17, wherein the substrate is selected from a 3-cyanopyridine, 8-hydroxyquinoline glucopyranoside, 8-hydroxyquinoline glucuronide, 8-hydroxyquinoline β-D-galactopyranaside, pyridoxal phosphate, and pyridoxamine phosphate.

19. The kit of Claim 17 or 18, wherein the catalyst is selected from peroxidase, nitrile hydratase, glucuronidase, glucosidase, galactosidase, and phosphatase.

20. The kit of any one Claims 17 to 19, wherein the catalyst is attached to a binder selected from an antibody, an aptamer, an affibody, and a ligand.
